# EUROPEAN PATENT SPECIFICATION

(11) **EP 1 420 787 B1**
(45) Date of publication and mention of the grant of the patent: **23.12.2009**
(21) Application number: 02767419.1
(22) Date of filing: 22.08.2002
(51) Int. Cl.: A61K 31/445, A61K 47/32, A61K 47/34, A61K 9/00, A61K 31/435

(54) **OPHTHALMIC COMPOSITION COMPRISING AN ASCOMYCIN**
ASCOMYCIN ENTHALTENDE OPHTHALMISCHE ZUBEREITUNGEN
COMPOSITIONS OPHTALMIQUES

(30) Priority: 23.08.2001 US 314448 P
(43) Date of publication of application: 26.05.2004
(73) Proprietor: Novartis AG, 4056 Basel (CH); Novartis Pharma GmbH, 1230 Wien (AT)
(72) Inventor: WONG, Michelle, Pik-han, Duluth, GA 30096 (US); MINICK, Kasey, Jon, Morrisville, NC 27560 (US); BABIOLE SAUNIER, Maggy, F-68130 Jettingen (FR); BIZEC, Jean-Claude, F-68440 Habsheim (FR); FETZ, Andrea, CH-8620 Wetzikon (CH); SCHOCH, Christian, CH-4132 Muttenz (CH)
(74) Representative: de Weerd, Petrus G.W.
(86) International application number: PCT/EP2002/009408
(87) International publication number: WO 2003/017990

(56) References cited:
- EP-A- 0 551 626
- WO-A-96/13249
- WO-A-99/34830
- LIN H-R ET AL: "Carbopol/pluronic phase change solutions for ophthalmic drug delivery" JOURNAL OF CONTROLLED RELEASE, ELSEVIER SCIENCE PUBLISHERS B.V. AMSTERDAM, NL, vol. 69, no. 3, 3 December 2000 (2000-12-03), pages 379-388, XP004221288 ISSN: 0168-3659
- ZIGNANI M ET AL: "TOPICAL SEMI-SOLID DRUG DELIVERY: KINETICS AND TOLERANCE OF OPHTHALMIC HYDROGELS" ADVANCED DRUG DELIVERY REVIEWS, AMSTERDAM, NL, vol. 16, no. 1, 1995, pages 51-60, XP000653437 ISSN: 0169-409X

## Description

This invention relates to ophthalmic compositions, e.g. gels, comprising an ascomycin or a compound of the FK 506 class for once-a-day administration.

We have found that ophthalmic compositions have often to be applied typically two to four times a day and that such repeated administration is not optimal in practice, e.g. for optimal treatment the patient has to have the medicament always available and the patient is disturbed several times a day. Such multiple administration of a drug, in particular of an ophthalmic composition, leads generally to the problem of overdosing and/or underdosing. Overdosing may typically generate ocular irritation, whereas underdosing may typically lead to reoccurrence of the symptoms.

There is thus a need for a so-called once-a-day administration of ophthalmic drugs.

FK506 is a known macrolide antibiotic that is produced by Streptomyces tsukubaensis No 9993. It is also a potent immunosuppressant. The structure of FK506 is given in the appendix to the Merck Index, 11th Edition as item A5. Methods of preparing FK506 are described in EP 184162. A large number of derivatives, antagonists, agonists and analogues of FK506, which retain the basic structure and at least one of the biological properties (for example immunological properties) of FK506, are known. These compounds are described in a large number of publications, for example EP 184162, EP 315978, EP 323042, EP 423714, EP 427680, EP 465426, EP 474126, WO 91/13889, WO 91/19495, EP 484936, EP 532088, EP 532089, EP 569337, EP 626385, WO 93/5059 and the like. Ascomycins and derivatives thereof, including FK506, are referred to hereinafter as "ascomycins".

Preferred ascomycins for use in the present invention include FK506; 33-epi-chloro-33-desoxy-ascomycin as disclosed in Example 66a in EP 427680 (hereinafter referred to as Compound A); {[1E-(1R,3R,4R)]1R,4S,5R,6S,9R,10E,13S,15S,16R,17S,19S,20S}-9-ethyl-6,16, 20-trihydroxy-4-[2-(4-hydroxy-3-methoxy-cyclohexyl)-1-methylvinyl]-15,17-dimethoxy-5,11,13,19-tetramethyl-3-oxa-22-aza-tricyclo[18.6.1.0(1,22)]heptacos-10-ene-2,8,21,27-tetraone as disclosed in Examples 6d and 71 in EP 569 337; and {1R,5Z,9S,12S-[1E-(1R,3R,4R)],13R,14S,17R,18E,21S,23S,24R,25S,27R}17-ethyl-1,14-dihydroxy-12-[2-(4-hydroxy-3-methoxy-cydohexyl)-1-methylvinyl]-23,25-dimethoxy-13,19,21,27-tetramethyl-11,28-dioxa-4-aza-tricyclo(22.3.1.0(4,9)]octacosa-5,18-diene-2,3,10,16-tetraone, also known as 5.6-dehydro-ascomycin as disclosed in Example 8 in EP 626 385. Particularly preferred is Compound A.

We have found that compositions comprising an ascomycin may be formulated for once-a-day administration which provide a therapeutic effect of the ascomycin at the eye over 24 hours and such compositions are surprisingly well tolerated. Moreover said once-a-day compositions produce a highly reliable and more reproducible clinical result in a patient treated therewith.

Therefore, in one aspect the present invention provides a stabilized aqueous ophthalmic composition being adapted for topical once-a-day administration to the eyes consisting of an ascomycin (hereinafter compositions of the present invention) and at least one polymer selected from
i) a polyoxyethylene-polyoxypropylene co-polymer or block co-polymer; and
ii) an acrylic acid homo- or co-polymer, wherein said acrylic acid homo or co-polymer is crosslinked, and a tonicity ennancing agent, a buffer and a preservative.

The concentration of an ascomycin is preferably from about 0.005 - 3.0%, more preferably from 0.01 - 1.5by weight based on the total weight of the composition. If desired, the compositions of the present invention may be in the form of a suspension, e.g. containing particles of an ascomycin e.g. with a mean particle diameter of 0.2 to 900 nm.

Applicants have found compositions of the present invention with moderate viscosity, e.g. from 50 to 2000, e.g. about 100 to 1000, mPa s at 20-25°C, which are comfortable to apply. Upon instillation into the eye, the viscosity of the compositions of the present invention may increase or decrease. Preferably the viscosity decreases upon instillation into the eye. The compositions of the present invention still have an excellent retention after instillation into the eye.

The compositions of the present invention may comprise pharmaceutically acceptable exciplents, which are suitable for ophthalmic compositions. For example the excipients and/or compositions of the present invention should not significantly affect the lacrimal system nor the ocular tear film.

information on the properties, specifications and characteristics are described e.g in standard texts such as Fiedler, H.P.; 1996; Lexikon der Hilfsstoffe für Pharmazie, Kosmetik und angrenzende Gebiete; Editio Cantor Verlag Aulendorf (Germany), and Kibbe, A.H.; 2000; Handbook of Pharmaceutical Excipients, a joint publication of Pharmaceutical Press, London (UK), and American Pharmaceutical Association, Washington (US) as well as manufacturers' brochures, the contents of which are incorporated herein by reference.

The compositions of the present invention contains
1.1 polyoxyethylene-polyoxypropylene copolymers and preferably block co-polymers. Preferably the polyoxypropylene polymer number is from about 10 to about 60. Preferably the polyoxyethylene polymer number is from about 10 to about 150. Examples include such as those known and commercially available under the trade names Lutrol® and Poloxamer® (Fiedler, loc. cit., p. 1200. 1203; Handbook of Pharmaceutical Excipients, loc. cit., page 386) and in particular Poloxamer® 407 and Lutol® F127, having a melting point of about 52 to 57°C; and
1.2 acrylic acid homo- and co-polymers, which are cross-linked; preferably carboxypolymethylene. Preferred molecular weights are between about 500,000, preferably from 1,000,000 to about 10,000,000 Daltons. Preferably, the acid groups comprise between 56 and 68% by weight of the total polymer.
   Preferably it is crosslinked with a polyol, e.g.
   1.2.1 with divinyl glycol, such as those known and commercially available under the trade name Noveon® from BFGoodrich, and in particular Noveon® AA-1, or
   1.2.2 with allylsucrose or allypentaerythritol, such as those known and comercially available under the trade name Carbopol® from BFGoodrich. Examples include those known and commercially available under the trade name Carbopol® and in particular Carbopol® 934P,974P,980,981 and 984.

The exact amounts of polymer/thickener components may vary within wide limits, e.g. to produce a composition of the present invention within the viscosity Indicated above. For example the amount may be from e.g. 0.05 to 10%, by weight of the total composition.

The present invention provides ophthalmic compositions comprising an ascomycin and at least one polymer such as i) a polyoxyethylene-polyoxypropylene co-polymer or block co-polymer, and ii) a cross-linked acrylic acid polymer, e.g. as hereinabove described, e.g. adapted for topical once-a-day administration to the eye.

Preferably both polymer/thickener components component i) and ii) are present e.g. in a weight ratio of i):ii) of e.g. 1:200 to 1:5, e.g. 1:50 to 1;20.

The compositions of the present invention further contains (2.) a tonicity enhancing agent Suitable tonicity enhancing agents are, e.g.
- 2.1: ionic compounds, such as alkali metal or alkaline earth metal halides, such as CaCl₂, KBr, KCl, LiCl, Nal, NaBr or NaCl, or boric acid, and/or
- 2.2: non-ionic compounds such as urea, glycerol, sorbitol, mannitol, propylene glycol, or dextrose.

Conveniently, sufficient tonicity enhancing agent is added to impart to the ready-for-use ophthalmic composition an osmolality of approximately from 50 to 1000 mOsmol, preferred from 100 to 400 mOsmol, more preferred from 200 to 400 mOsmol and even more preferred from 280 to 350 mOsmol.

For the adjustment of the pH, preferably to a physiological pH, the composition of the present invention contains (3.) a pharmaceutically acceptable buffer system. Examples of buffer substances are acetate, ascorbate, borate, hydrogen carbonate/carbonate, citrate, gluconate, lactate, phosphate, propionate and tromethamine (tris-(hydroxymethyl)-aminomethane, TRIS) buffers. Tromethamine buffer is preferred. The buffer substance added is typically of an amount to ensure and maintain a physiologically tolerable pH range. The pH range is generally in the range of from 4 to 9, preferably from 4.5 to 8.5 and more preferably from 5.0 to 8.2.

The compositions of the present invention further contains (4.) a preservative, e.g. on storage or to inhibit microbial growth after opening a closed container holding such a composition and exposing such a composition to the air. A preservative may typically be selected from e.g.
- 4.1: a quaternary ammonium compound such as e.g. benzalkonium chloride (N=benzyl-N-(C₈-C₁₈-alkyl)-N,N-dimethylammonium chloride), benzoxonium chloride, cetrimide (hexadecyl-trimethylammonium bromide) or the like.
- 4.2: alkyl-mercury salts of thiosalicylic acid, such as e.g. thiomersal, phenylmercuric nitrate, phenylmercuric acetate or phenylmercuric borate,
- 4.3: parabens, such as e.g. methylparaben or propylparaben,
- 4.4: alcohols, such as e.g. chlorobutanol, benzyl alcohol or phenyl ethanol,
- 4.5: biguanide derivatives, such as e.g. chlorohexidine or polyhexamethylene biguanide,
- 4.6: sodium perborate,
- 4.7: imidazolidinyl urea as known and commercially available under the trade name Germal®II,
- 4.8: sorbic acid,
- 4.9: stabilized oxychloro complexes such as known and commercially available under the trade name Purite®,
- 4.10: polyglycol-polyamine condensation resins, such as known and commercially available e.g. under the trade name Polyquart® from Henkel KGaA, and/or
- 4.11: a mixture of any components 4.1 to 4.10.

Preferred preservatives are quaternary ammonium compounds, in particular benzalkonium chloride and cetrimide. Where appropriate, a sufficient amount of preservative is added to the ophthalmic composition to ensure protection against secondary contaminations during use caused by bacteria and fungi, e.g. the preferred preservatives are present in an amount of about 0.001-0.02%.

A pharmaceutical composition may additionally require the presence of (5.) a solubilizer, . A solubilizer suitable for an above concerned composition is e.g.
- 5.1: octylphenoxy-poly(ethylenoxy)-ethanol (tyloxapol) known and commercially available under the trade name Triton@, e.g. Triton ® WR 1339, (Fiedler, loc. cit., p 1609),
- 5.2: polyethylene glycol glyceryl fatty acid ester. The fatty acid ester may include mono and/or di and/or tri fatty acid ester. The fatty acid constituent may include both saturated and unsaturated fatty acids having a chain length of from e.g. C₈-C₂₀. The polyethylene glycols may have e.g. from 5 to 40 [CH₂-CH₂-O] units, e.g. 5 or 30 units. Particularly suitable is polyethylene glycol (15) glyceryl monostearate or polyethylene glycol (15) glyceryl monooleate which is commercially available, e.g. under the trade name TGMS®-15 or TGMO®-15, respectively, e.g. from Nikko Chemicals Co., Ltd. Further suitable is polyethylene glycol (30) glyceryl monooleate which is commercially available, e.g. under the trade name Tagat® O, e.g. from Goldschmidt (H. Fiedler, loc cit, vol. 2, p. 1502-1503). Further suitable are polyethylene glycol glyceryl C₈-C₁₀ fatty acid ester with from 5 to 10 [CH₂-CH₂-O] units, e.g. 7 units, e.g. Cetiol® HE, or Labrasol®
- 5.3: polyoxyethylene C₈₋₂₀ fatty acid esters, e.g. polyoxyethylene stearic acid esters of the type known and commercially available under the trade name Myrj® (Fiedler, loc. cit., 2, p. 1042) or Brij® (Fiedler, loc. cit., p. 259; Handbook of Pharmaceutical Excipients, loc. cit., p. 367). An especially preferred product of this class is Myrj® 52 having a D²⁵ of about 1.1, a melting point of about 40 to 44°C, an HLB value of about 16.9, an acid value of about 0 to 1 and a saponification value of about 25 to 35,
- 5.4: glycerol ethers (Fiedler, loc. cit., p.701),
- 5.5: cyclodextrins, e.g. α-, β- or γ-cyclodextrin, e.g. alkylated, hydroxyalkylated, carboxyalkylated or alkyloxycarbonyl-alkylated derivatives, or mono- or diglycosyl-α-, β- or γ-cyclodextrin, mono- or dimaltosyl-α-, β- or γ- cyclodextrin or panosyl-cyclodextrin, e.g. such as known and commercially available under the trade name Cavamax® or Cavasol® from Wacker Chemie. An especially preferred product of this class is hydroxypropyl- y-cyclodextrin, e.g. as known and commercially available under the trade name Cavasol® W7 HP or Cavasol® W8 HP. A mixture of cyclodextrins may also be used.
- 5.6: polyoxyethylene-sorbitan- C₈₋₂₀ fatty acid esters (polysorbates) e.g. produced by co-polymerising ethylene oxide with fatty acid esters of a sorbitol and its anhydrides of e.g. mono- and tri- lauryl, palmityl, stearyl and oleyl esters e.g. of the type known and commercially available under the trade name Tween® (Fiedler, loc.cit., p.1615) including the products Tween®
20 [polyoxyethylene(20)sorbitanmonolaurate],
21 [polyoxyethylene(4)sorbitanmonolaurate],
40 [polyoxyethylene(20)sorbitanmonopalmitate],
60 [polyoxyethylene(20)sorbitanmonostearate],
65 [polyoxyethylene(20)sorbitantristearate],
80 [polyoxyethylene(20)sorbitanmonooleate],
81 [polyoxyethylene(5)sorbitanmonooleate),
85 [polyoxyethylene(20)sorbitantrioleate]. Especially preferred products of this class are Tween®20 and Tween®80.
- 5.7: reaction products of natural or hydrogenated vegetable oils and ethylene glycol, i.e. polyoxyethylene glycolated natural or hydrogenated vegetable oils, for example poly-oxyethylene glycolated natural or hydrogenated castor oils. Such products may be obtained in known manner, e.g. by reaction of a natural or hydrogenated castor oil or fractions thereof with ethylene oxide, e.g. in a molar ratio of from about 1:35 to about 1:60, with optional removal of free polyethylene glycol components from the product, e.g. in accordance with the methods disclosed in German Auslegeschriften 1,182,388 and 1,518,819. Especially suitable are the various tensides available under the trade name Cremophor. Particularly suitable are the products Cremophor RH 40 having a saponification no. ca. 50-60, an acid no.=<1, an iodine no.=<1, a water content (Fischer)=<2%, an n_{D}⁶⁰ =ca.1,453-1,457 and an HLB=ca. 14-16; Cremophor RH 60 having a saponification no.=ca. 40-50, an acid no. =<1, an iodine no.=<1, a water content (Fischer)=ca. 4.5-5.5%, an n_{D}²⁵=ca.1.453-1,457 and an HLB=ca.15-17; and Cremophor EL having a molecular weight (by steam osmometry)=ca. 1630, a saponification no.=ca. 65-70, an acid no.=ca. 2, an iodine no.=ca. 28-32 and an n_{D} ²⁵ =ca.1.471 (c.f. Fiedler loc. cit. p. 326-327). Also suitable for use in this category are the various tensides available under the trade name Nikkol, e.g. Nikkol HCO-60. The said product NIKKOL HCO-60 is a reaction product of hydrogenated castor oil and ethylene oxide exhibiting the following characteristics: acid no.=ca. 0.3; saponification no.=ca. 47.4; hydroxy value=ca. 42.5. pH (5%)=ca. 4.6; Color APHA=ca. 40; m.p.=ca. 36.0°C.; Freezing point=ca. 32.4°C.; H₂Ocontent (%, KF)=ca. 0.03, and/or
- 5.8: mixtures of the components 5.1 to 5.7.

Especially preferred solubilizers are Cremophor EL, Cremophor RH 40, tyloxapol and cyclodextrins. The concentration used depends especially on the concentration of the active ingredient. The amount added is typically sufficient to solubilize the active ingredient. For example, the concentration of the solubilizer is from 0.1 to 5000 times the concentration of the active ingredient, preferably 0.5 to 1000, e.g. 1 to 500.

Further excipients may be comprised in the compositions of the present invention, which may in particular function as a combined stabilizer/solubilizer. Such a combined additional stabilizer/solubilizer is for example a cyclodextrin or a mixture of cyclodextrins. A preferred cyclodextrin is in particular selected from the group of α-cyclodextrin, β-cyclodextrin, γ-cyclodextrin, hydroxypropyl-β-cyclodextrin, hydroxypropyl-γ-cyclodextrin, dimethyl-β-cyclodextrin, randomly methylated β-cyclodextrin and dimethyl-γ-cyclodextrin. The amount is generally in the range of from approximately 0.01 to approximately 90% by weight, more preferably in the range of from 0.1 - 20% by weight.

The ophthalmic compositions may comprise further pharmaceutically acceptable excipients, such as (6.) emulsifiers, (7.) wetting agents or (8.) fillers, such as, e.g. the polyethylene glycols (Fiedler, loc. cit., p. 2108, Handbook of Pharmaceutical Excipients, loc. cit., p 392) such as PEG 200, 300, 400 and 600, or Carbowax® 1000, 1500, 4000, 6000 and 10000.

Other excipients that may be used if desired are listed below but they are not intended to limit in any way the scope of the possible excipients. They are especially (9.) complexing agents, such as disodium-ethylenediamine tetraacetate, ethylenediamine tetraacetic acid (EDTA), (10.) antioxidants, such as ascorbic acid, acetylcysteine, cysteine, sodium hydrogen sulfite, butylated hydroxyanisole, butylated hydroxytoluene or alpha-tocopherol acetate; (11.) stabilizers, such thiourea, thiosorbitol, sodium dioctyl sulfosuccinate or monothioglycerol; or (12.) other excipients, such as, for example, lauric acid sorbitol ester, triethanol amine oleate or palmitic acid ester. Preferred exipients are complexing agents, such as disodium-EDTA. The amount and type of excipient added is in accordance with the particular requirements and is generally in the range of from about 0.0001 to about 90% by weight.

In another embodiment, the present invention provides for compositions further comprising (13.) an ophthalmic carrier. Such carriers are typically adapted for topical administration, and are for example
- 13.1: water,
- 13.2: mixtures of water and water-miscible solvents, such as C₁- to C₇-alkanols,
- 13.3: vegetable oils or mineral oils comprising from 0.5 to 5% by weight hydroxyethyl-cellulose, ethyl oleate, carboxymethyl-cellulose, polyvinyl-pyrrolidone,
- 13.4: water-soluble polymers for ophthalmic uses, such as, for example, cellulose derivatives, such as methylcellulose, alkali metal salts of carboxy-methylcellulose, hydroxymethylcellulose, hydroxyethylcellulose, methylhydroxypropyl-cellulose and hydroxypropylcellulose,
- 13.5: acrylates or methacrylates, such as salts of polyacrylic acid or ethyl acrylate, poly-acrylamides,

- 13.6: natural products, such as gelatin, alginates, pectins, tragacanth, karaya gum, gellan gum such as Gelrite®, xanthan gum, carrageenin, agar and acacia,
- 13.7: starch derivatives, such as starch acetate and hydroxypropyl starch,
- 13.8: synthetic products, such as polyvinyl alcohol, polyvinylpyrrolidone, polyvinyl methyl ether, polyethylene oxide, or
- 13.9: mixtures of those polymers.
Preferred carriers are water, cellulose derivatives, such as methylcellulose, alkali metal salts of carboxymethylcellulose, hydroxymethylcellulose, hydroxyethylcellulose, methylhydroxy-propylcellulose and hydroxypropylcellulose, or mixtures thereof. The concentration of the carrier is, for example, from 1 to 100 000 times the concentration of the active ingredient.

It will be appreciated that although the excipients have been described above by reference to a particular function any particular excipient may have alternative or multiple functions, e.g. cyclodextrin or a mixture of cyclodextrins may act as e.g. stabilizer, complexing agent and/or solubilizer.

The compositions of the present invention are surprisingly stable, as indicated by conventional tests, e.g. under stressed conditions, such as 15h at 80°C or 1 month at 40°C. The compositions of the present invention are stable over 2, even 3, years showing less than 5 % degradation of the ascomycin at 20 to 30°C. This surprising stability is also observed in an aqueous composition comprising an ascomycin. Such aqueous compositions represent the preferred ophthalmic compositions of the present invention and contain an ascomycin typically in suspension.

A typical example of an aqueous ascomycin suspension contains:

| **1% Suspension** | **%** | **mg/mL** |
|---|---|---|
| 33-epi-chloro-33-desoxy-ascomycin. (Micronised) | 1% | 10 mg/mL |
| Pluronic F127 NF (Poloxamer 407) | 0.1% | 1.0 mg/mL |
| Carbopol 934P NF | 0.2% | 2.0 mg/mL |
| Mannitol, USP | 4.3% | 43 mg/mL |
| Benzalkonium Chloride | 0.015% | 0.15mg/mL |
| Sodium Hydroxide | *Adjust to pH 6.5 | *Adjust to pH 6.5 |
| Water for injection | qs 400g | qs 400g |

The present invention is therefore also useful to stabilize an aqueous composition comprising an ascomycin, in particular comprising an ascomycin suspension.

The ophthalmic compositions of the present invention may be prepared in conventional manner e.g. by mixing an ascomycin and appropriate excipients.

Filling may be effected before or after sterilization of the resulting mixture. Sterilization of the composition of the present invention and the primary package can be effected e.g. by gamma irradiation, by ethylene oxide treatment, by electron beam, by autoclaving, by microwave treatment, by filtration through a sterile filter, or by steam sterilization.

The compositions of the present invention may be packaged in conventional manner. The compositions of the present invention may be stored in single or multiple unit dosage form, e.g. closed bottles, tubes or other containers made from glass, plastic such as e.g. polyethylene, polyethylene terephthalate, or polypropylene, or metal or combinations thereof. For example bottles may contain about 1 to 10 ml of the compositions of the present invention. The container may be fitted with a dropper to facilitate administration.

The compositions of the present invention may be formulated in conventional manner e.g. to be particularly adapted for topical ophthalmic use. In so far as the procedures for formulation are not particularly described herein such formulation procedures may for example be known in the art, or analogous to those known in the art or to procedures described herein. Representative procedures are disclosed in for example, Remington's Pharmaceutical Sciences, 19th Ed., Mack Publ., Co., 1995, H. Sucker et al, Pharmazeutische Technologie, 2nd Edition, Thieme, 1991, R: H. Mueller et al, Pharmazeutische Technologie: Moderne Arzneimittelformen, 2nd Edition, Wissenschaftliche Verlagsgesellschaft, Stuttgart, 1998, L. Lachman et al. The Theory and Practice of Industrial Pharmacy, 3rd Ed, 1986, and Hagers Handbuch der pharmazeutischen Praxis, 4th Ed. Vol. 7, (Springer Verlag, 1971) as well as later editions, the contents of all of which are incorporated herein by reference.

The excipients used may e.g. be those known in the art e.g in the Lexikon der Hilfsstoffe für Pharmazie, Kosmetik und angrenzende Gebiete; and Handbook of Pharmaceutical Excipients, references referred to above, or analogous to those known in the art or new excipients having analogous function to those described in the art or herein.

The compositions of the present invention are useful for the treatment of immune-mediated conditions of the eye, such as auto-immune diseases, e.g. dry eye, uveitis, keratoplasty or chronic keratitis; allergic conditions, e.g. vernal conjunctivitis; inflammatory conditions or corneal transplants, and in particular of dry eye, and may be used for the treatment and prevention of signs and symptoms of the ocular conditions as indicated e.g. in standard animal trials and clinical trials.

One animal test comprises a modified Draize test on three albino rabbits wherein the ocular tolerability after a single dose instillation of 50 microlitres of compositions of the present invention on the ocular surface is shown for the 15 minutes after instillation then after 1, 2 and 7 days. The tolerability was based on visual examination considering the following parameters: discomfort as judged by blinking or partial/complete closure of the eye, duration of discomfort, discharge, redness of conjunctiva (palpebral and bulbar conjunctiva), chemosis of conjunctiva (swelling), degree of opacity of cornea and area of cornea involved, and pathological influence upon iris.

A clinical trial may be effected to test the efficacy and tolerability of about 30 to 40 microlitre of compositions of the present invention containing 1% of an ascomycin administered once a day by instillation onto the ocular surface, e.g. to the inside lower lid, to groups of, e.g. 10 to 25, healthy volunteers, or patients suffering from allergic conjunctivitis. The trial lasts e.g. 8 days.

The subjects are examined to determine the effect against conjunctivitis, e.g. fast onset of action and long duration of action and good tolerability, e.g. lack of significant irritation or reddening.

Additionally the bioavailability of the compositions of the present invention in the above trials may be determined by absorption in the conjunctiva or surrounding tissues.

The bioavailability of an addressed once-a-day ophthalmic composition may be assessed with the pharmacokinetic assay described infra:
A fixed volume, e.g. 50 microliters, of the ophthalmic formulation was instilled onto the upper part of the conjunctiva of rabbits. Tears, bulbar conjunctiva, cornea and sclera were sampled after either 5, 15, 30 minutes, or, 1, 8, 16, or 20 h. Samples were extracted for ascomycin determination related to the wet weight amount of tissue or tears. Ascomycin was determined using a liquid chromatography linked to mass spectrography (LC-MS) validated method.

The retention of an addressed once-a-day ophthalmic composition may be assessed with retention tests described infra:
The upper eyelid of one eye of an albino rabbit is carefully pulled away from the eyeball and 50 microliter of the test substance is instilled on the superior part of the bulbar conjunctiva using a gauged automatic pipette. The eyelid is gently closed for about one second. After either 5 min, 1 hour, 4 hours, 8 hours, 16 hours or 24 hours a weighed Schirmer's test strip is maintained in the cul-de-sac between the inferior lid and the temporal part of the eyeball for exactly one minute. The absorbed tears collected on each Schirmer test strip are immediately weighed and the ascomycin content is determined e.g. after extracting the strips by liquid chromatography/ negative ion chemical ionization mass spectrometry using a deuterated analogue of the ascomycin as internal standard. The strips may be kept frozen up to analytical test of the ascomycin content.

The exact amount of the ascomycin to be administered will naturally depend on a variety of factors, e.g. choice of salt, excipients, formulation properties, and severity of the condition. Conveniently, the composition of the present invention is administered to the cornea once a day, e.g. after breakfast. Preferably from about 25 to about 75 microlitres, e.g. from about 50 to about 75 microlitres, is administered, e.g. using a dropper.

The daily dose of the ascomycin to be administered is from about 1 micrograms/kg to about 5 micrograms/kg. For larger mammals, e.g. a 70 kg mammal such as a human, a dose of from about 100 to about 300 micrograms, is indicated.

Therefore, in a further aspect the present invention provides
a) an ophthalmic composition as defined above for use in the treatment of immune-mediated conditions of the eye, such as auto-immune diseases, e.g. dry eye, uveitis, keratoplasty or chronic keratitis; allergic conditions, e.g. vernal conjunctivitis; inflammatory conditions or corneal transplants, in particular dry eye, or a condition treatable by ascomycin therapy, or
   the use of a composition of the present invention in the preparation of a medicament for the treatment of immune-mediated conditions of the eye, such as auto-immune diseases, e.g. dry eye, uveitis, keratoplasty or chronic keratitis; allergic conditions, e.g. vernal conjunctivitis; inflammatory conditions or corneal transplants, in particular dry eye, or a condition treatable by ascomycin therapy.

All percentages referred to herein are weight/weight except where otherwise indicated.

## Claims

1. A stabilized aqueous ophthalmic composition adapted for topical once-a-day administration to the eye consisting of an ascomycin and at least one polymer selected from
i) a polyoxyethylene-polyoxypropylene co-polymer or block co-polymer,and
ii) an acrylic acid homo- or co-polymer wherein said acrylic acid homo- or co-polymer is crosslinked; and a tonicity enhancing agent, a buffer, and a preservative.

2. A composition according to claim 1 further comprising an ophthalmic carrier.

3. A composition according to any preceding claim wherein the ascomycin is 33-epi-chloro-33-desoxy-ascomycin.

4. Use of a composition according to anyone of claims 1 to 3 in the preparation of a medicament for the treatment of immune-mediated conditions of the eye, such as auto-immune diseases, e.g. dry eye, uveitis, keratoplasty or chronic keratitis; allergic conditions, e.g. vernal conjunctivitis; inflammatory conditions or corneal transplants or another condition treatable by ascomycin therapy.

5. Use according to claim 4 in the preparation of a medicament for the treatment of dry eye.

## Patentansprüche

1. Stabilisierte wässrige ophthalmische Zusammensetzung, die für eine topische Verabreichung einmal pro Tag an das Auge angepasst ist und die aus einem Ascomycin und mindestens einem Polymer, das aus
i) einem Polyoxyethylen-Polyoxypropylen-Copolymer oder -Blockcopolymer und
ii) einem Acrylsäurehomo- oder -copolymer, wobei das Acrylsäurehomo- oder -copolymer vernetzt ist, ausgewählt ist und einem die Tonizität erhöhenden Mittel, einem Puffer und einem Konservierungsmittel besteht.

2. Zusammensetzung nach Anspruch 1, die ferner einen ophthalmischen Träger umfasst.

3. Zusammensetzung nach einem der vorhergehenden Ansprüche, wobei das Ascomycin 33-Epichlor-33-desoxyascomycin ist.

4. Verwendung einer Zusammensetzung nach einem der Ansprüche 1 bis 3 zur Herstellung eines Medikaments zur Behandlung von immunologischen Zuständen des Auges, wie Autoimmunerkrankungen, z.B. trockenem Auge, Uveitis, Keratoplastie oder chronischer Keratitis, allergischen Zuständen, z.B. Frühjahrs-Konjunktivitis, Entzündungszuständen oder Hornhauttransplantaten oder anderen durch Ascomycintherapie behandelbaren Zuständen.

5. Verwendung nach Anspruch 4 bei der Herstellung eines Medikaments zur Behandlung von trockenem Auge.

## Revendications

1. Composition ophtalmique aqueuse stabilisée adaptée à une seule administration locale quotidienne dans l'oeil, constituée d'une ascomycine et d'au moins un polymère choisi parmi
i) un copolymère ou copolymère séquencé de polyoxyéthylène-polyoxypropylène, et
ii) un homo- ou copolymère d'acide acrylique, lequel homo- ou copolymère d'acide acrylique est réticulé ; et un agent d'amélioration de la tonicité, un tampon, et un conservateur.

2. Composition selon la revendication 1, comprenant en outre un véhicule ophtalmique.

3. Composition selon l'une quelconque des revendications précédentes, dans laquelle l'ascomycine est la 33-épi-chloro-33-désoxy-ascomycine.

4. Utilisation d'une composition selon l'une quelconque des revendications 1 à 3 dans la préparation d'un médicament destiné au traitement d'affections à médiation immunologique de l'oeil telles que les maladies auto-immunes, par exemple la sécheresse oculaire, l'uvéite, la kérotoplastie ou la kératite chronique ; d'affections allergiques, par exemple la conjonctivite printanière ; d'affections inflammatoires ou de greffes de cornée ou d'une autre affection pouvant être traitée par un traitement à base d'ascomycine.

5. Utilisation selon la revendication 4 dans la préparation d'un médicament destiné au traitement de la sécheresse oculaire.
